# EUROPEAN PATENT SPECIFICATION

(11) **EP 2 391 268 B1**
(45) Date of publication and mention of the grant of the patent: **30.09.2020**
(21) Application number: 10706167.3
(22) Date of filing: 27.01.2010
(51) Int. Cl.: A61B 5/053, G01R 27/02

(54) **APPARATUS FOR DIAGNOSING A DISEASED CONDITION IN TISSUE OF A SUBJECT**
VORRICHTUNG ZUR DIAGNOSE EINER ERKRANKUNG IM GEWEBE EINES SUBJEKTS
APPAREIL DE DIAGNOSTIC D'UN ÉTAT MALADE DANS LE TISSU D'UN SUJET

(30) Priority: 27.01.2009 US 147617 P
(43) Date of publication of application: 07.12.2011
(73) Proprietor: Scibase Ab, 103 67 Stockholm (SE)
(72) Inventor: ÅBERG, Peter, 122 46 Enskede (SE)
(74) Representative: Groth & Co. KB
(86) International application number: PCT/EP2010/050922
(87) International publication number: WO 2010/086326

(56) References cited:
- EP-A2- 1 959 828
- WO-A1-2005/074796
- WO-A2-01/56468
- WO-A2-01/64095
- ABERG PETER ET AL: "Assessment of skin lesions and skin cancer using simple electrical impedance indices" SKIN RESEARCH AND TECHNOLOGY, MUNKSGAARD, COPENHAGEN, DK LNKD- DOI:10.1034/J.1600-0846.2003.00017.X, vol. 9, no. 3, 1 January 2003 (2003-01-01) , pages 257-261, XP009136093 ISSN: 0909-752X [retrieved on 2003-07-25]
- EMTESTAM L ET AL: "Electrical Impedance of Nodular Basal Cell Carcinoma: a pilot study" DERMATOLOGY, S. KARGER AG, CH LNKD- DOI:10.1159/000018023, vol. 197, no. 4, 1 January 1998 (1998-01-01), pages 313-316, XP008026174 ISSN: 1018-8665
- OLLMAR S ET AL: "INFORMATION IN FULL AND REDUCED DATA SETS OF ELECTRICAL IMPEDANCE SPECTRA FROM VARIOUS SKIN CONDITIONS, COMPARED USING A HOLOGRAPHIC NEURAL NETWORK" MEDICAL AND BIOLOGICAL ENGINEERING AND COMPUTING, SPRINGER, HEILDELBERG, DE, vol. 35, no. 4, 1 July 1997 (1997-07-01), pages 415-419, XP000658159 ISSN: 0140-0118

## Description

### FIELD OF THE INVENTION

The present invention generally relates to the diagnosis, determination, characterization or assessment of biological conditions, particularly diseased conditions, in tissue of a human or animal subject. More particularly, the present invention is related to diagnosing of a diseased condition, for example skin cancer, such as basal cell carcinoma or malignant melanoma, in tissue of such a subject, by employing tissue electrical impedance data.

### BACKGROUND

Skin cancer is a rapidly increasing form of cancer in many countries throughout the world. The most common form of skin cancers are basal cell carcinoma, squamous cell carcinoma, and melanoma. Melanoma is one of the rarer types of skin cancer but causes the majority of skin cancer related deaths. It has been suggested that the majority of skin cancer cases are caused by too much exposure to sunlight. As with other types of cancer, it is important that skin cancer, especially melanoma, is diagnosed at such an early stage as possible.

However, clinical diagnosis of skin tumours may prove difficult even for experienced dermatologists, especially in the case of malignant melanoma. Thus, there is an increasing need for a diagnostic aid besides the established method of employing ocular inspections in combination with skin biopsies for histological examination.

As known in the art, electrical impedance constitutes a very sensitive indicator of changes in organic and biological material, especially in tissues such as mucous membranes, skin and integuments of organs, and may thus provide an effective tool for noninvasive measurements of variations in structural properties of organic and biological material. Therefore, a lot of effort has been made to find a simple and reliable way to measure variations and alterations in organic and biological material, in order to establish the occurrence of such variations and alterations which are due to different states, characteristics or irritations from abnormal conditions, such as diseased conditions. A number of invasive, micro-invasive and noninvasive techniques for determining biological conditions employing electrical impedance measurements or spectra have accordingly been proposed in the art.

In general, it is desirable to determine diseased conditions in tissue at such an early stage of disease development as possible, when preventive measures may easily be effectuated and/or are more effective compared to when applied at a later stage in the episode of disease development.

Specifically, skin cancer, especially malignant melanoma, may very rapidly spread into adjoining tissue and organs, thus making it very dangerous, even possibly fatal, if diagnosed at a late stage in the disease development. Other types of skin cancer, such as basal cell carcininoma and squamous cell carcinoma, are less likely to spread to other parts of the body, even if malignant. However, they may be locally disfiguring if not treated early. Like many cancers, skin cancers generally start as precancerous lesions, which may initially be quite small. In this regard, clinical experience has shown that lesions, especially in early stages, may include very small malignant parts, having malignant foci smaller than 1 mm in diameter. It is therefore very desirable to be able to diagnose even small-sized tumours at an early stage in the disease.

Electrical impedance imaging has been proposed to form an image of electrial impedance differences within a body region. It is noted that the image does not necessarily need to correspond to an actual image of an abnormal condition, e.g., a lesion, but may rather be construed broadly as a pattern that may be used for identifying such abnormal conditions. However, the separation of diseased tissue, such as malignant tumors, from healthy tissue or merely mildly diseased tissue (e.g., benign lesions) based on impedance measurements needs further investigation. In this regard, there are fundamental problems that need to be addressed when trying to construct an image or pattern from impedance data. For one thing, electrical currents within the body follow the path of least resistance, in general being a irregular path not restricted to a particular line or even a plane in the body, which may be an issue in reconstructing the spatial distribution of electrical properties in the body from impedance data. Furthermore, electrical impedance data obtained from impedance measurements in tissue is multivariate and further comprises complex numbers, comprising magnitude and phase. Notwithstanding the problem of analyzing complex numbers, such multivariate data further generally represents a very large data set which may be cumbersome to analyze, even with powerful data processing means.

Conventional methods and devices for diagnosing diseased conditions within tissues and internal organs of subjects generally lack versatile means for being able to accurately and efficiently map a large set of multivariate impedance data with a diseased condition that may be present. It would therefore be desirable with improved algorithms for processing multivariate impedance data for the purpose of identifying any diseased condition that may be present.

Devices for diagnosing diseased conditions of tissue are known from WO 01/64095 A2, XP009136093, and EP 1 959 828 A2.

Further to this, it has been demonstrated that conventional approaches for measurements of variations in structural properties of organic and biological material, particularly determination of small malignant regions of diseased tissue, generally lack the required accuracy and/or structural resolution (tissue resolution) due to a limited resolution in the measured impedance spectra for detecting and/or characterizing minutely sized abnormalities in the tissue, such as small-sized lesions.

Thus, there is a need in the art for an improved scheme or algorithm for diagnosing of diseased conditions of the tissue of a subject, particularly skin, which provides a versatile means for accurately and efficiently processing a large set of multivariate impedance data in order to identify any diseased condition that may be present. Also, it would be desirable with an improved scheme capable of providing an increased accuracy and/or tissue resolution compared to conventional approaches. In particular, it would be desirable with an improved technique for diagnosing of skin cancer, for example malignant melanoma, basal cell carcinoma, squamous cell carcinoma or precursors thereof, that provides an increased accuracy and/or structural resolution compared to conventional approaches.

### SUMMARY OF THE INVENTION

In view of the above, it is an object of the present invention to provide an improved method and medical apparatus for diagnosing a diseased condition in tissue of a subject, by employing electrical impedance data measured in the tissue.

This and other objects are completely or partially achieved by an apparatus for diagnosing a diseased condition in tissue of a subject according to the independent claims. Further embodiments are defined by the dependent claims.

It is contemplated that the present invention may be applied both to human subjects and to subjects of other animals.

According to an aspect of the present invention, there is provided a medical apparatus according to claim 1.

By such an apparatus, there is achieved similar or identical advantages to the advantages of the method according to the first aspect of the present invention.

According to an embodiment of the present invention, the diagnosing unit is adapted to perform the trained evaluation system algorithm for diagnosis of the diseased condition in the target tissue region further on the basis of the classified data set for the reference tissue region.

According to the present invention, impedance data of the target tissue region and/or the reference tissue region are/is obtained at different tissue layers, wherein at least an upper portion of the tissue is scanned so as to obtain a series of impedance values from small consecutive tissue partitions.

Thereby, it is possible to obtain impedance data having a high degree of resolution. This allows for detection of small anomalies in the tissue. The resolution is, in principle, limited by the distance between adjacent electrodes used for obtaining the impedance data, the frequency of the alternating currents, and the overall design of the electrodes (for example, the size and shape of the electrodes).

Further, tissue impedance may be measured at different tissue layers, in general a plurality of different tissue layers, which may be arranged in a series from the topmost layer to the lowermost layer included in the measurement.

Thereby, a high tissue resolution with respect to the depth below the tissue surface may be achieved by making the distance between measurement points in adjacent tissue layers small, the resolution in principle being limited only by how small a distance between measurement points in adjacent tissue layers that may be realized.

According to another embodiment of the present invention, the noise content in the impedance data of the target tissue region and/or the impedance data of the reference tissue region is reduced. The process of reducing the noise content in the impedance data may comprise one or more of the following: differentiating at least one of the plurality of impedance values of the target tissue region and/or the reference tissue region with respect to time, space, phase and/or magnitude, determining the magnitude, the phase, the real part, and/or the imaginary part of at least one of the plurality of impedance values of the target tissue region and/or the reference tissue region, determining the difference between at least one of the plurality of impedance values of the target tissue region and at least one of the plurality of impedance values of the reference tissue region, and determining the reciprocal of at least one of the plurality of impedance values of the target tissue region and at least one of the plurality of impedance values of the reference tissue region. Thus, the accuracy of the diagnosing may be even further increased due to the removal of, e.g., biological noise in the impedance data.

According to yet another embodiment of the present invention, the dimensionality of the impedance data of the target tissue region and/or the impedance data of the reference tissue region is reduced. This may be performed by means of linear reduction, for example by Principal Component Analysis (PCA), of the impedance data of the target tissue region and/or the impedance data of the reference tissue region, or non-linear reduction, for example by non-linear Kernel PCA, of the impedance data of the target tissue region and/or the impedance data of the reference tissue region. Alternatively or optionally, the dimensionality reduction may be performed by means of Cole-Cole equivalent circuit modelling, self-organizing map, and impedance indexing. It is to be understood that this exemplary list is not exhaustive. The dimensionality reduction may also be performed by means of a combination of two or more techniques such as, but not limited to, the ones mentioned immediately above.

In general, the so obtained impedance data sets comprise a very large number of variables, which implies that it may be ambiguous to perform univariate analysis of each variable (analysis of one variable at a time) because of information redundancy. By the embodiment of the present invention, the data may be simplified to a smaller number of variables but still contain the clinically relevant information, thus allowing for quicker and more powerful analysis or processing of the impedance data for subsequent diagnosis of diseased conditions in tissue.

According to yet another embodiment not forming part of the present invention, data on the subject's physical conditions is received and at least some of the data is parameterized, wherein the diagnosing of the diseased condition in the target tissue region on the basis of the classified data set for the target tissue region and the classified data set for the reference tissue region, by performing the trained evaluation system algorithm, is done further on the basis of the thus parameterized data on the subject's physical conditions. The data on the subject's physical conditions may include one or more of the subject's age, lesion ABCDE characteristics, the subject's gender, lesion size, location of the lesion and the subject's erythema susceptibility.

In this manner, the diagnosing of the diseased condition is further performed on the basis of additional clinically relevant data, and thus, the accuracy of the diagnosis of the diseased condition may be even further improved. Such additional data may be directed to diagnosing special kinds of diseased conditions, such as skin cancer (for which the above-listed data may be especially relevant). Hence, by inclusion of specially selected additional data on the subject's physical conditions, particular diseased conditions may be targeted. According to an exemplary embodiment of the present invention, the method and/or apparatus are/is specifically arranged for diagnosing skin cancer, such as basal cell carcinoma or malignant melanoma, or precursors thereof, such as for example acitinic keratose (a precursor of squamous cell carcinoma) and dysplastic nevi (a precursor of malignant melanom), or conditions of the skin comprising age, sun damage and collagen composition.

A wide range of classifiers may be applied for pattern recognition, ranging from simple linear classifiers to very powerful artificial networks.

Hence, according to yet another embodiment not forming part of the present invention, the at least one set of data pre-processing rules are determined by, e.g., one or more of Fisher Linear Discriminant, Partial Least Squares Discriminant Analysis, k-Nearest Neighbors, Support Vector Machines, Artificial Neural Networks, Bayesian Classifiers and decision trees.

According to yet another embodiment not forming part of the present invention, at least one set of data pre-processing rules are applied to the parameterized data on the subject's physical conditions, the rules being determined for example by one or more of Fisher Linear Discriminant, Partial Least Squares Discriminant Analysis, k-Nearest Neighbors, Support Vector Machines, Artificial Neural Networks, Bayesian Classifiers and decision trees, thus classifying also the parameterized data on the subject's physical conditions which may enhance the performance of the diagnosis of the diseased condition of tissue.

According to yet another embodiment of the present invention, the impedance data of the target tissue region and/or the reference tissue region are/is obtained at a plurality of frequencies between about 10 Hz and about 10 MHz and/or at a plurality of different current drive amplitudes.

Earlier studies have demonstrated that in order to accurately obtain indications of diseased conditions, a significant portion of the frequency spectrum, for example 10 Hz to 10 MHz, may have to be taken into account in the analysis of impedance data (see, for example, figure 2(a)-2(d) in EP 1600104 A1). Furthermore, by considering impedance data obtained at a plurality of different current drive amplitudes, it is for example possible to detect departures from linear response, which may be used to indicate when local potential variations in the tissue at cellular level become large enough to exceed cellular potential variations, hence allowing new ionic conduction paths to be utiliized. Such nonlinearities normally do not occur in healthy tissue, and thus the above approach may be useful in further increasing the performance of diagnosing diseased conditions in tissue.

Acording to the invention, the electrical impedance of the target tissue region and/or the reference tissue region is measured at a plurality of logarithmically distributed frequencies ranging from about 1 kHz to about 2.5 MHz, for instance at 35 logarithmically distributed frequencies. According to this particular example, ten measurement frequencies per decade are used.

According to yet another embodiment not forming part of the present invention, the trained evaluation system is selected from, for example, a neural network, an expert system and a combination thereof.

In the context of the present invention, by the term "substantially concurrently or immediately consecutively" it is meant, for example, that obtaining the impedance data for the target tissue region and obtaining the impedance data for the reference tissue region may take place possibly during the same time or with only such a short time interval between such that the measurement process is practically feasible, possibly dependent on the particular configuration of the probe that is used. This has the advantage that it ensures that obtaining the impedance data for the target tissue region and obtaining the impedance data for the reference tissue region are carried out under quite similar external conditions so as not to introduce any artefacts in the so obtained impedance data of the target tissue region and reference tissue region.

In the context of the present invention, by the term "lesion" it is meant a tumour of the skin.

In the context of the present invention, by the term "ABCDE criteria" it is meant criteria for assessing if a mole on a subject might be suspected of malignant melanoma, namely asymmetry (A) with respect to the borders from one side of the mole to the other, border irregularity (B) with respect to jaggedness of the borders of the mole or if the color at the border of the mole is not uniform, color (C) as in multiple colors occuring in a single mole, diameter (D) of the mole, for example if the diameter of the mole exceeds about 6 mm, and evolution (E) of the mole, that is change in shape, size or color with time.

In the context of the present invention, by the term "ABCDE characteristics" it is meant a tissue region of a subject or patient, such as a mole, a lesion, etc., characterized according to the above-mentioned ABCDE criteria.

Other objectives, features and advantages of the present invention will appear from the following detailed disclosure, from the attached claims as well as from the drawings.

Generally, all terms used in the claims are to be interpreted according to their ordinary meaning in the technical field, unless explicitly defined otherwise herein. All references to "a/an/the [element, device, component, unit, means, step, etc]" are to be interpreted openly as referring to at least one instance of said element, device, component, unit, means, step, etc., unless explicitly stated otherwise. The steps of any method disclosed herein do not have to be performed in the exact order disclosed, unless explicitly stated.

### BRIEF DESCRIPTION OF THE DRAWINGS

The above, as well as additional objects, features and advantages of the present invention, will be better understood through the following illustrative and non-limiting detailed description of preferred embodiments of the present invention, with reference to the appended drawings, where the same reference numerals are used for identical or similar elements, wherein:
Figure 1 is a schematic view of a medical apparatus according to an exemplary embodiment of the present invention;
Figure 2 is a schematic view of a probe for measuring tissue impedance according to an exemplary embodiment of the present invention;
Figures 3-5 are schematic views of medical apparatuses according to exemplary embodiments of the present invention; and
Figure 6 is a schematic flowchart illustrating a method for diagnosing a diseased condition in tissue of a subject according to an exemplary embodiment of the present invention.

### DETAILED DESCRIPTION OF PREFERRED EMBODIMENTS

In the preceding and in the following, various operations are described as a multiple of discrete steps that are performed in turn in a manner helpful for understanding the present invention. However, the order of description should not be construed as to imply that these steps are necessarily performed in the order in which they are presented, or even dependent on the order in which they are presented.

In general, so-called "raw" electrical impedance data obtained from electrical impedance measurements in tissue is multivariate and further comprises complex numbers, comprising magnitude and phase, or real and imaginary parts. In order to interpret such raw data it is therefore desirable and often necessary to fit the data in a model or simplify the data into a manageable number of clinically relevant parameters. For instance, the impedance data may be processed for reducing the number of variables by linear projections of the impedance data to lower subspaces. For this purpose, techniques such as principal component analysis (PCA) may be used. Alternatively, non-linear projections of the impedance, for example by non-linear Kernel PCA, may be used. Further alternative approaches include parallel factor analysis (PARAFAC), Cole-Cole equivalent circuit modelling, Self-organizing maps, or simple impedance indices. Such techniques are known in the art and detailed description thereof is therefore omitted. The thus simplified data may be further processed by, for example, classical statistical analysis or classification.

Numerical classification of electrical impedance and a diseased condition (e.g., a lesion) in the tissue of a subject may be used to provide means for finding rules that describe the relationship between the electrical impedance and identity of the diseased condition (the lesion) as well as further characteristics, for example directed at whether a lesion is malignant. Such rules may then be employed for identifying the diseased condition (the lesion) and/or characterize another non-identified diseased condition (a lesion) using impedance measurements. For this purpose, the rules must first be adjusted using training sets, namely impedance measurements of both benign and diseased conditions (lesions) with known identity and/or characteristics (for instance determined clinically by ocular inspection in combination with tissue biopsies for histological analysis). It is to be understood that "adjustment" of the rules is to be construed broadly, in that it may comprise modifying the numerical values of the parameters of a particular classification rule, or classification model, or even changing the classification rule (or model) itself.

In the context of diagnosing diseased condition, e.g. lesions, the performance of the classifiers generally needs to be validated, for example by comparing electrical impedance measurements of new lesions which does not have been included in any training set used for training the classification rules. An electrical impedance measurement of a new lesion, for which the identity and/or characteristics have been established (for example clinically by ocular inspection in combination with lesion biopsies for histological analysis), comprising a so called test set, after the classification rules have been determined is a reliable way of validating the classification rules. Furthermore, this procedure closely mimics the intended use of the classifier. The performance of the classifier is then approximated using the relation between the established identities and/or characteristics of the subsets and the predicted identities and/or characteristics of the subsets using the classifier. It is to be understood that the same procedure may be applied in the context of any diseased condition.

As used in the foregoing and in the following, by "classification rules" it is meant data processing rules for processing data such as to classify the data.

According to exemplary embodiments not forming part of the present invention, classification rules determined by means of, for example, one or more of Fisher Linear Discriminant (FLD), Partial Least Squares Discriminant Analysis (PLS-DA), Soft Independent Modelling of Class Analogy (SIMCA), k-Nearest Neighbors (KNN), Support Vector Machines (SVM) Artificial Neural Networks (ANN), decision trees and Bayesian classifiers are used, as further described in the following. Such techniques for determining classification rules are known in the art and detailed description thereof is therefore omitted.

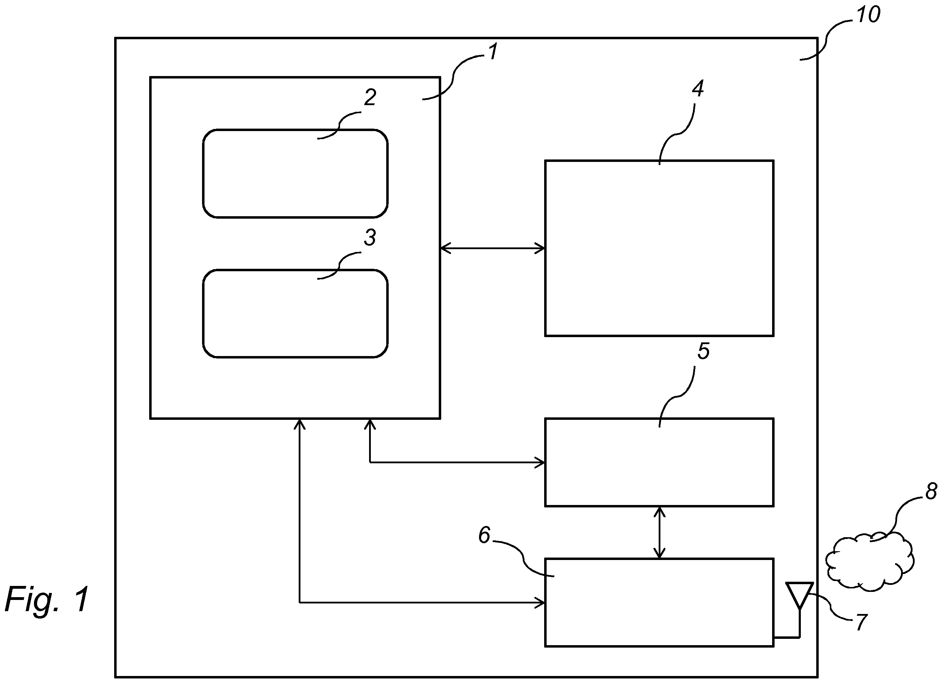

Figure 1 is a schematic view of a medical apparatus 10 for diagnosing a diseased condition in tissue of a subject according to an exemplary embodiment of the present invention. The apparatus 10 comprises a main unit 1 for performing the core operations of the apparatus, the main unit 1 including a impedance signal unit 2 and a classifying unit 3. The main unit 1 is connected to a diagnosing unit 4 for diagnosing the diseased condition in the tissue on the basis of impedance data obtained by the impedance signal unit 2.

The impedance signal unit 2 is adapted to obtain impedance data of a target tissue region of the tissue of the subject and to obtain impedance data of a reference tissue region of the tissue of the subject. It is to be understood that the impedance data of the target tissue region comprises a plurality of impedance values measured in the target tissue region, and the impedance data of the reference tissue region comprises a plurality of impedance values measured in the reference tissue region. According to an exemplary embodiment, the tissue of the subject comprises skin of the subject. However, the method and apparatus as described herein could equally well be applied to a tissue biopsy (test sample) or to a point under the skin of a subject (subcutaneously), by means of, e.g., sharp, pointed electrodes allowing for insertion under the skin. By the target tissue region it is meant a tissue region which is to be diagnosed, namely a tissue region which is suspected of being afflicted by a diseased condition. By the reference tissue region it is meant a tissue region used for reference purposes and which is in a healty state. The reference tissue region should generally be arranged such that it is located in close proximity to the target tissue region, or at least as close as possible while still allowing for distinct electrical impedance measurements to be carried out.

According to the invention, the impedance signal unit 2 is adapted to obtain the impedance data of the target tissue region and the impedance data of the reference tissue region substantially concurrently or immediately consecutively. By this it is meant that the measurements for obtaining the respective impedance data sets are performed substantially concurrently or immediately consecutively. The target and reference tissue surfaces may be soaked prior to each impedance measurement using for example a 0.9 % saline solution. For instance, the surfaces may be soaked for about 30 seconds prior to the electrical impedance measurements being carried out.

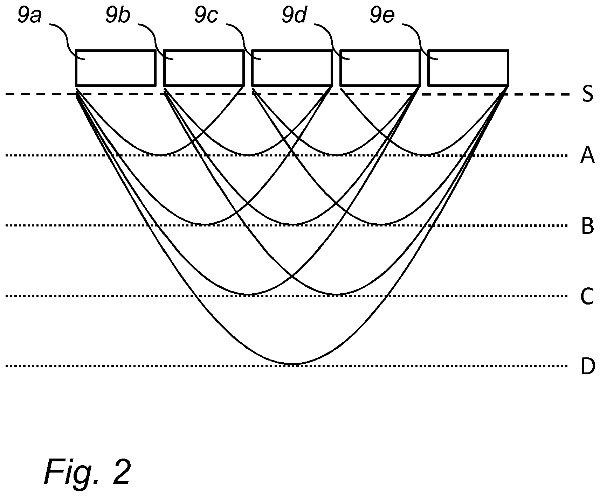

The tissue impedance measurements for obtaining the impedance data of the target tissue region and/or the reference tissue region may be performed by means of a probe integrated in the medical apparatus 10 or a probe being external to the medical apparatus 10 and connected to the medical apparatus 10. For example, irrespective of being external or integrated, the probe may comprise a plurality of electrodes adapted to be placed in contact with the tissue to be analysed, typically skin of the subject. The tissue impedance may be measured by applying an AC voltage over two electrodes and measuring the resulting current. Such an electrode probe according to the invention comprises five electrodes arranged to substantially cover a tissue surface area when the probe is placed in contact with the tissue. By selecting adjacent pairs of electrodes, the topmost layer of the tissue can be scanned by the resulting current path. By selecting electrode pairs that are not adjacent, or in other words, electrode pairs having one or more intermediately arranged electrodes, the resulting current paths allow for scanning (measuring) at deeper tissue layers. This is illustrated in figure 2, wherein a number of current paths are indicated, as well as a number of tissue layers A, B, C and D, schematically indicated by dotted lines. The surface S of the tissue is schematically indicated by the dashed line. By in turn applying a voltage over pairs of adjacent electrodes (voltage applying means are not shown), that is electrodes 9a and 9b, 9b and 9c, 9c and 9d, or 9d and 9e, four impedance measurements can be made in the topmost layer A of the tissue by measuring the resulting current paths. In this manner, a scan of the topmost tissue layer A can be done. Similarly, by in turn applying a voltage over pairs of electrodes having one intermediately arranged electrode, that is electrodes 9a and 9c, 9b and 9d, or 9c and 9e according to the illustrated exemplary case, three impedance measurements may be made in a tissue layer B immediately below the topmost tissue layer A by measuring the resulting current paths. Similarly, by in turn applying a voltage over pairs of electrodes having two intermediately arranged electrodes, namely electrodes 9a and 9d and 9b and 9e, two impedance measurements can be made in the tissue layer C immediately below the tissue layer B by measuring the resulting current paths. According to the embodiment illustrated in figure 2, a final measurement can also be made at a still deeper tissue layer D, immediately below the tissue layer C, by applying a voltage over pairs of electrodes having three intermediately arranged electrodes, according to this particular example the electrodes 9a and 9e, and measuring the resulting current path. By such configurations, electrical impedance at even deeper tissue depths may be measured.

Furthermore, by moving the probe along the tissue surface and performing impedance measurements at a plurality of locations, a lateral scan may be made relatively the tissue surface.

Turning again to figure 1, the impedance data for the target tissue region and the reference tissue region thus aquired are subsequently classified by the classifying unit 3 which is adapted to apply at least one set of classification rules to the impedance data of the target tissue region and to the reference tissue region so as to obtain a classified data set for the target tissue region and a classified data set for the reference tissue region.

The thus classified data sets are then inputted to the diagnosis unit 4, which subsequently, on the basis of the classified data set for the target tissue region and the classified data set for the reference tissue region, performs a trained evaluation system algorithm for diagnosing the diseased condition in the target tissue region. The result of the diagnosis may then be outputted to the user (clinician), for example by means of suitable visual display means, such as a LCD panel, or a printer. It is also contemplated that the result of the diagnosis may be transmitted to an external device (not shown), for example the clinician's laptop or stationary computer, to be digitally stored thereon. The apparatus may be provided with a separate communication unit for this purpose, capable of communicating with external devices via a wireless communications medium or via electrical conductors. Also, according to the embodiment illustrated in figure 1, the result of the diagnosis may also be transmitted to the external device by means of an integrated communication unit 6 (as indicated by the double arrow in figure 1), further described in the following. It is to be understood that the double arrows in figure 1 indicate that communication between the respective components may be two-way.

The set of classification rules applied to the impedance data of the target tissue region and to the impedance data of the reference tissue region may for example be determined by one or more of Fisher Linear Discriminant, Partial Least Squares Discriminant Analysis, k-Nearest Neighbors, Support Vector Machines, Artificial Neural Networks, Bayesian Classifiers and decision trees. Such techniques are known in the art and detailed description thereof is therefore omitted. It is the purpose that such techniques may be combined with any of the embodiments described in the foregoing and in the following.

The impedance signal unit is further adapted to obtain impedance data of the target tissue region and/or the reference tissue region at different layers in the tissue, wherein at least a topmost tissue layer of the tissue to be analysed is scanned, that is electrical impedance at a point in the tissue pertaining to at least a topmost layer is measured, so as to obtain a series of impedance values from the topmost tissue layer. In other words, tissue impedance is measured at points in the tissue pertaining to different tissue layers, in general a plurality of different tissue layers, which may be arranged in a series from the topmost layer to the lowermost layer included in the measurement. This may for example be carried out according to the previous description associated with figure 2. Such measurement of tissue impedance may also be carried out employing one or more of the apparatuses described in the co-pending application published under WO 2010/085969 A1 by the same applicant, entitled "Switch probe for multiple electrode measurement of impedance". In this way, a high tissue resolution with respect to the depth below the tissue surface may be achieved, by making the distance between measurement points in adjacent tissue layers small. The resolution that can be achieved is in principle limited only by how small a distance between measurement points in adjacent tissue layers that may be realized in the apparatus. It is the purpose that a configuration such as described immediately above may be combined with any one of the embodiments described in the foregoing and in the following.

As illustrated in figure 1, the medical apparatus 10 may further include a processing unit 5 adapted to reduce the noise content in the impedance data of the target tissue region and/or the impedance data of the reference tissue region. Alternatively or optionally, the processing unit 5 may also be adapted to reduce the dimensionality of the impedance data of the target tissue region and/or the impedance data of the reference tissue region.

For the purpose of reducing the noise content in the impedance data of the target tissue region and/or the impedance data of the reference tissue region, the processing unit 5 may be further adapted to differentiate at least one of the plurality of impedance values of the target tissue region and/or the reference tissue region with respect to time, space, phase and/or magnitude. Alternatively or optionally, the processing unit 5 may be further adapted to determine the magnitude, the phase, the real part, and/or the imaginary part of at least one of the plurality of impedance values of the target tissue region and/or the reference tissue region. Furthermore, alternatively or optionally, the processing unit 5 may be further adapted to determine the difference between at least one of the plurality of impedance values of the target tissue region and at least one of the plurality of impedance values of the reference tissue region. Also, alternatively or optionally, the processing unit 5 may be further adapted to determine the reciprocal of at least one of the plurality of impedance values of the target tissue region and at least one of the plurality of impedance values of the reference tissue region. Thus, the accuracy of the medical apparatus 10 may be further increased due to the removal of, e.g., biological noise in the thus aquired impedance data.

Any one of the medical apparatuses 10 in the embodiments described in the foregoing and in the following may comprise a processing unit 5 such as described immediately above and elsewhere herein.

As illustrated in figure 1, the apparatus may further comprise a communication unit 6 capable of transmitting/receiving data to/from an external device (not shown), which may be a laptop computer, a handheld computer, a database, etc. In this way, the apparatus may be supplied with, for example, data for facilitating the diagnosis of the diseased condition of the tissue, as will be described in the following. For example, the communication unit 6 may be adapted to receive data on the subject's physical conditions, which may include, but not be limited to, the subject's age, lesion ABCDE characteristics, the subject's gender, lesion size, location of the lesion and the subject's erythema susceptibility. The processing unit 5 may be adapted to parameterize at least some of the thus received data, inputted from the communication unit 6 to the processing unit directly or via the main unit 1, as illustrated in figure 1. Then, the diagnosing unit 4 may perform the trained evaluation system algorithm for diagnosis of said diseased condition in the target tissue region further on the basis of the parameterized data on the subject's physical conditions.

The communication unit 6 may be arranged to transmit/receive data via a wireless communications medium or via electrical conductors ("wires") connected between the communication unit 6 and the external device. As illustrated in figure 1, for this purpose the communication unit 6 may comprise an antenna 7 adapted to communicate with external devices (not shown) via a wireless communications network 8. It is further to be understood that communications may be performed such that they are protected from third party tampering, as well known in the art.

Any one of the medical apparatuses 10 in the embodiments described in the foregoing and in the following may comprise a communication unit 6 such as described immediately above and elsewhere herein.

According to the present invention, a trained evaluation system algorithm is employed to diagnose diseased conditions in the tissue of the subject. By training the at least one set of classification rules, the performance (accuracy) of the trained evaluation system algorithm may be improved. A trained evaluation system algorithm, non-limiting examples of which are expert systems and/or neural networks, is generally employed to identify and learn the signature pattern of different tissue types or conditions, including cancerous and precancerous tissues, within the multivariate data comprising a plurality of impedance values as measured in the target tissue region and/or the reference tissue region. In order for the trained evaluation system algorithm to identify and learn the signature pattern of different tissue types and conditions, it may use a pattern-recognition algorithm that identifies regions within the multivariate data space corresponding to different tissue types and conditions. For accurate and reliable operation of the method and apparatus according to the embodiments of the present invention, the trained evaluation system algorithm must be capable of making accurate and reliable evaluations based on the classified data set for the target tissue region and/or the reference tissue region, which is ensured by training the at least one set of classification rules used for classifying the impedance data of the target tissue region and/or the reference tissue region. For this purpose, the classification rules may be gradually adjusted using training sets and subsequently validated, in such a manner as has been previously described.

It is to be understood that the present invention is not limited to training only the classification rules, but the performance (accuracy) of the trained evaluation system algorithm may also be improved by training the algorithm architecture, namely the combination of in general different classification rules and/or other data processing steps as described in the foregoing. Such training may be performed analogously to the training of the classification rules.

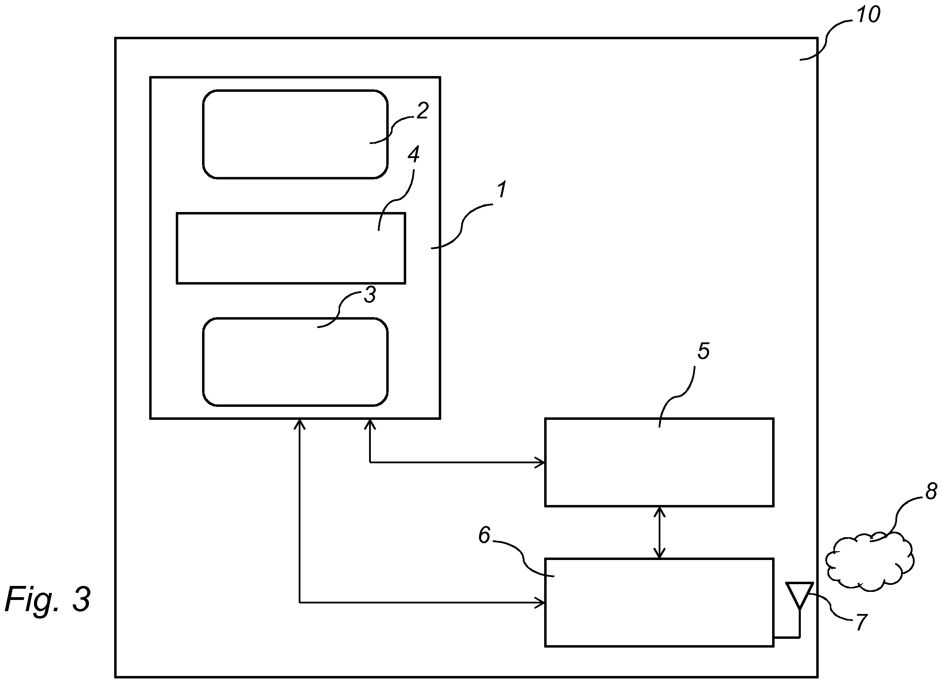

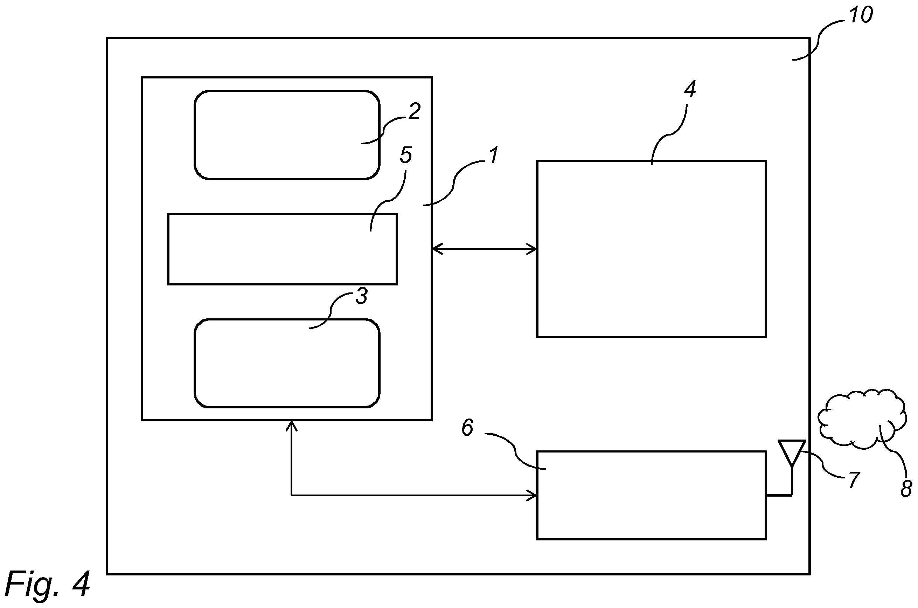

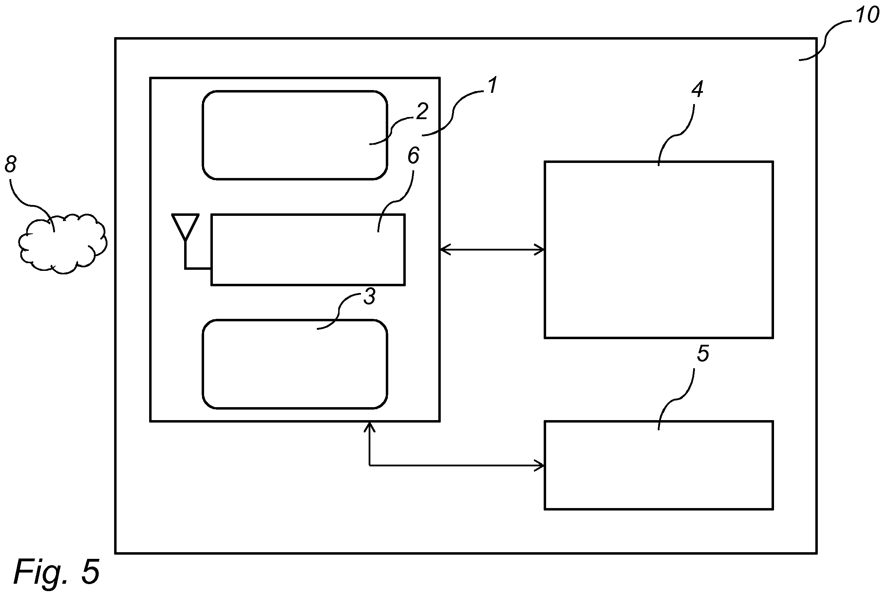

Figures 3-5 are schematic views of medical apparatuses 10 for diagnosing a diseased condition in tissue of a subject according to three exemplary embodiments of the present invention. The medical apparatuses 10 illustrated in figures 3-5 are in many respects similar to the medical apparatus 10 described with reference to figure 1. However, as illustrated in figure 3, the main unit 1 of the medical apparatus 10 may further comprise a diagnosing unit 4. Thus, according to the illustrated embodiment in figure 3, the diagnosing unit 4 may be integrated in the main unit 1. Furthermore, as illustrated in figure 4, the main unit 1 of the medical apparatus 10 may optionally comprise a processing unit 5, thus being integrated in the main unit 1. Alternatively or optionally, it is also contemplated that the main unit 1 of the medical apparatus 10 may comprise a communication unit 6, as illustrated in figure 5. It is also contemplated that the main unit 1 in other embodiments may comprise a diagnosing unit 4, a processing unit 5 and/or a communication unit 6. Thus, one or more of the diagnosing unit 4, the processing unit 5 and the communication unit 6 may be integrated in the main unit 1 of the medical apparatus 10.

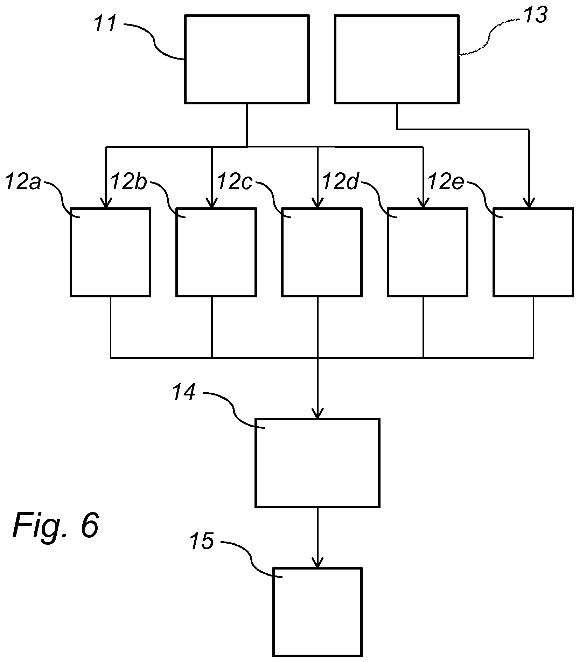

Figure 6 is a schematic flowchart illustrating a method for diagnosing a diseased condition in tissue of a subject according to an exemplary embodiment not forming part of the present invention. The method begins by obtaining impedance data of the tissue of the subject. At step 11, impedance data of the target tissue region, which data comprises a plurality of impedance values measured in the target tissue region, and impedance data of the reference tissue region, which data comprises a plurality of impedance values measured in the reference tissue region, are obtained. As previously described, the reference tissue region is preferably such that it is located in close proximity to the target tissue region. Furthermore, the impedance data of the target tissue region and the impedance data of the reference tissue region are obtained substantially concurrently or immediately consecutively, in such a way that the measurements of the tissue impedance in the target tissue region and in the reference tissue region are performed substantially concurrently or immediately consecutively, as has been previously described above.

As illustrated in figure 6, the method continues with steps 12a-12d, each of which comprises applying a set of classification rules to the thus obtained impedance data of the target tissue region and/or the reference tissue region. In general, the sets of classification rules applied in each of the steps 12a-12d may be different from one another. Each one of steps 12a-12d may further comprise further processing of the impedance data, preferably prior to the classification procedures being carried out. Such processing may include reduction of the noise content in the impedance data of the target tissue region and/or the reference tissue region, and/or reduction of the dimensionality of the impedance data of the target tissue region and/or the reference tissue region, as has been previously described in the foregoing.

As illustrated in figure 6, the method may further include a step 13 comprising receiving data on the subject's physical conditions and parameterizing at least some of the thus received data on the subject's physical conditions. The thus parameterized data on the subject's physical conditions may subsequently be classified and/or further processed at step 12e, similarly to steps 12a-12d.

The method continues at step 14, comprising diagnosing of the diseased condition in the target tissue region on the basis of the classified data sets for the target tissue region and the classified data sets for the reference tissue region obtained at steps 12a-12d and the classified parameterized data on the subject's physical conditions obtained at step 12e by applying the trained evaluation system algorithm. It is to be understood that steps 13 and 12e are optional, and the diagnosing of the diseased condition in the target tissue region by applying the trained evaluation system algorithm may be performed on the basis of the classified data sets for the target tissue region and the classified data sets for the reference tissue region obtained at one or more of steps 12a-12d only.

The method ends at step 15 with having provided an outcome of the trained evaluation system algorithm, namely a diagnosis of a diseased condition in the tissue of the subject, to the user (e.g., a clinician or a dermatologist).

Human skin is a complex heterogenous and anisotropic multilayer structure having electronically non-linear properties. Particularly the Stratum Corneum (that is the outermost layer of the epidermis), below which diseases such as skin cancer and allergic reactions manifest, is characterized by highly non-linear effects and very high electrical impedance. Thus, depending on the particular design of the measurement probe, non-invasive electrical impedance spectra of skin may be dominated by the dielectric properties of the Stratum Corneum, especially at low frequencies. Furthermore, the Stratum Corneum has a large and broad so called alpha dispersion that may lead to that responses from underlying viable skin layers may be confounded with responses from the Stratum Corneum, thus diluting the clinically relevant information from the viable skin.

In order to improve the assessment of electrical impedance phenomena that manifest below the Stratum Corneum, according to the present invention, the impedance data is obtained by means of an electrically conducting probe having a plurality of electrodes, where each electrode of the plurality of electrodes comprises at least one spike or micro-needle, each spike or micro-needle having a sufficient length to penetrate at least one layer of the skin of a subject or having a sufficient length to penetrate below the surface of the skin of a subject to the deepest layer of the epidermis, the Stratum Germinativum. For example, each spike or micro-needle may have a length up to 1 mm for skin cancer assessments, whereas other tissues and organs, which may be encapsulated with a thicker envelope, may require longer spikes or micro-needles. Furthermore, each electrode of the plurality of electrodes may comprise at least two, three, four, five, six, seven, eight, nine, ten, twelve, fifteen, eighteen, twenty, twenty-two, thirty, forty or at least fifty such spikes or micro-needles. By such configurations, in addition to possibly alleviating the problem of non-linear effects of the Stratum Corneum, an increased versatility and an increased adaptability in terms of capacity requirements may be achieved. According to a further embodiment, the spikes or micro-needles of the probe are laterally spaced apart from each other, so as to not interfere with each other when impedance is measured by, e.g., applying a voltage over a pair of spiked or micro-needled electrodes and measuring the resulting current.

According to an exemplary embodiment, the probe comprises a plurality of micro-needles arranged on a base substrate, such as a silicon wafer. The fabrication of micro-needles extending from the plane of a silicon wafer is known in the art, see for example US 2004/0243063; S. Roy and A. J. Fleischman, "Microneedle array module and method of fabricating the same".

It is to be understood that in the context of the present invention and in relation to electrical components electrically connected to each other, the term connected is not limited to mean directly connected, but also encompasses functional connections having intermediate components. For example, on one hand, if an output of a first component is connected to an input of a second component, this comprises a direct connection. On the other hand, if an electrical conductor directly supplies a signal from the output of the first component substantially unchanged to the input of the second component, alternatively via one or more additional components, the first and second component are also connected. However, the connection is functional in the sense that a gradual or sudden change in the signal from the output of the first component results in a corresponding or modified change in the signal that is input to the second component.

In conclusion, the present invention relates to a medical device for diagnosing a diseased condition in tissue of a human or animal subject, wherein tissue electrical impedance measurements are employed. At least one set of data pre-processing rules are applied to impedance data of a target tissue region and impedance data of a reference tissue region, wherein the reference tissue region is located in close proximity to the target tissue region. The impedance data of the target tissue region and the impedance data of the reference tissue region comprises a plurality of impedance values measured in the target tissue region and the reference tissue region, respectively, wherein the tissue mesurements in the two tissue regions are performed substantially concurrently or immediately consecutively. On the basis of the pre-processed data, a trained evaluation system algorithm diagnoses the diseased condition in the target tissue region.

The present invention has mainly been described above with reference to a few embodiments. However, as is readily appreciated by a person skilled in the art, other embodiments than the ones disclosed above are equally possible within the scope of the present invention, as defined by the appended claims.

## Claims

1. A medical apparatus (10) for diagnosing a diseased condition of tissue of a subject, including:
an impedance signal unit (2) being coupled to a probe comprising a plurality of electrodes adapted to obtain impedance data of a target tissue region at different tissue layers,
wherein the probe has five electrodes (9a - 9e), where each electrode (9a - 9e) comprises at least one spike or micro-needle, each spike or micro-needle having a sufficient length to penetrate at least one layer of the skin of a subject and wherein said impedance data comprises a plurality of impedance values measured in the target tissue region, at different tissue layers, the impedance signal unit (2) being further adapted to obtain impedance data of a reference tissue region being in close proximity to the target tissue region, wherein the impedance signal unit (2) is further adapted to obtain the impedance data of the target tissue region and the impedance data of the reference tissue region at different tissue layers substantially concurrently or immediately consecutively, wherein said impedance signal unit (2) is adapted to select electrode pairs comprising adjacent electrodes (9a - 9e) to scan impedance values at a topmost tissue layer and electrode pairs comprising electrodes separated by one or more intermediate electrodes (9a - 9e) to scan impedance values at deeper tissue layers substantially concurrently or immediately consecutively at both the target tissue region and the reference tissue region,
wherein the impedance signal unit (2) is further adapted to obtain impedance data of the target tissue region and/or the reference tissue region at different tissue layers, wherein at least an upper portion of the tissue is scanned so as to obtain a series of impedance values from small consecutive tissue partitions;
wherein electrical impedance of the target tissue region and/or the reference tissue region is measured at a plurality of logarithmically distributed frequencies ranging from about 1 kHz to about 2.5 MHz;
a classifying unit (3) adapted to apply at least one set of data pre-processing rules to the impedance data of the target tissue region and to the reference tissue region so as to obtain a classified data set for the target tissue region and a classified data set for the reference tissue region; and
a diagnosing unit (4) adapted to perform a trained evaluation system algorithm for diagnosis of said diseased condition in the target tissue region on the basis of the classified data set for the target tissue region.

2. The apparatus according to claim 1, wherein the diagnosing unit (4) is adapted to perform the trained evaluation system algorithm for diagnosis of said diseased condition in the target tissue region further on the basis of the classified data set for the reference tissue region.

3. The apparatus according to claim 1, further comprising a processing unit (5) adapted to:
reduce the noise content in the impedance data of the target tissue region and/or the impedance data of the reference tissue region; and/or
reduce the dimensionality of the impedance data of the target tissue region and/or the impedance data of the reference tissue region.

4. The apparatus according to claim 3, wherein the processing unit (5) is further adapted to:
differentiate at least one of the plurality of impedance values of the target tissue region and/or the reference tissue region with respect to time, space, phase and/or magnitude;
determine the magnitude, the phase, the real part, and/or the imaginary part of at least one of the plurality of impedance values of the target tissue region and/or the reference tissue region;
determine the difference between at least one of the plurality of impedance values of the target tissue region and at least one of the plurality of impedance values of the reference tissue region; and/or
determine the reciprocal of at least one of the plurality of impedance values of the target tissue region and at least one of the plurality of impedance values of the reference tissue region.

## Patentansprüche

1. Medizinische Vorrichtung (10) zur Diagnose einer Erkrankung in Gewebe eines Subjekts, welche einschließt:
eine Impedanzsignaleinheit (2), die an eine Sonde gekoppelt ist, welche eine Vielzahl von Elektroden umfasst, die vorgesehen sind, um Impedanzdaten einer Zielgeweberegion in unterschiedlichen Gewebeschichten zu erhalten,
wobei die Sonde fünf Elektroden (9a - 9e) aufweist, wobei jede Elektrode (9a - 9e) mindestens einen Dorn oder eine Mikronadel umfasst, wobei jeder Dorn oder jede Mikronadel eine ausreichende Länge aufweist, um mindestens eine Schicht der Haut eines Subjekts zu durchdringen, und wobei die Impedanzdaten eine Vielzahl von Impedanzwerten umfassen, die in der Zielgeweberegion in unterschiedlichen Gewebeschichten gemessen werden, wobei die Impedanzsignaleinheit (2) des Weiteren vorgesehen ist, um Impedanzdaten einer Referenzgeweberegion zu erhalten, die sich in enger Nähe zu der Zielgeweberegion befindet, wobei die Impedanzsignaleinheit (2) des Weiteren vorgesehen ist, um die Impedanzdaten der Zielgeweberegion und die Impedanzdaten der Referenzgeweberegion in unterschiedlichen Gewebeschichten im Wesentlichen gleichzeitig oder unmittelbar aufeinander folgend zu erhalten,
wobei die Impedanzsignaleinheit (2) vorgesehen ist, um Elektrodenpaare, die benachbarte Elektroden (9a - 9e) umfassen, um Impedanzwerte an einer obersten Gewebeschicht zu scannen, und Elektrodenpaare, die Elektroden umfassen, die durch eine oder mehrere Zwischenelektroden (9a - 9e) getrennt sind, um Impedanzwerte in tieferen Gewebeschichten zu scannen, auszuwählen, um im Wesentlichen gleichzeitig oder unmittelbar aufeinander folgend sowohl in der Zielgeweberegion als auch in der Referenzgeweberegion zu scannen,
wobei die Impedanzsignaleinheit (2) des Weiteren vorgesehen ist, um Impedanzdaten der Zielgeweberegion und/oder der Referenzgeweberegion in unterschiedlichen Gewebeschichten zu erhalten, wobei mindestens ein oberer Anteil des Gewebes so gescannt wird, dass eine Reihe von Impedanzwerten aus kleinen aufeinander folgenden Gewebepartien erhalten wird;
wobei die elektrische Impedanz der Zielgeweberegion und/oder der Referenzgeweberegion bei einer Vielzahl logarithmisch verteilter Frequenzen im Bereich von etwa 1 kHz bis etwa 2,5 MHz gemessen wird;
eine Klassifizierungseinheit (3), die vorgesehen ist, um mindestens einen Satz von Datenvorverarbeitungsregeln auf die Impedanzdaten der Zielgeweberegion und der Referenzgeweberegion anzuwenden, um so einen klassifizierten Datensatz für die Zielgeweberegion und einen klassifizierten Datensatz für die Referenzgeweberegion zu erhalten; und
eine Diagnoseeinheit (4), die vorgesehen ist, um einen trainierten Evaluierungssystemalgorithmus zur Diagnose der Erkrankung in der Zielgeweberegion basierend auf dem klassifizierten Datensatz für die Zielgeweberegion durchzuführen.

2. Vorrichtung nach Anspruch 1, wobei die Diagnoseeinheit (4) vorgesehen ist, um den trainierten Evaluierungssystemalgorithmus zur Diagnose der Erkrankung in der Zielgeweberegion des Weiteren basierend auf dem klassifizierten Datensatz für die Referenzgeweberegion durchzuführen.

3. Vorrichtung nach Anspruch 1, des Weiteren umfassend eine Verarbeitungseinheit (5), die vorgesehen ist zum:
Reduzieren des Rauschanteils in den Impedanzdaten der Zielgeweberegion und/oder den Impedanzdaten der Referenzgeweberegion; und/oder
Reduzieren der Dimensionalität der Impedanzdaten der Zielgeweberegion und/oder der Impedanzdaten der Referenzgeweberegion.

4. Vorrichtung nach Anspruch 3, wobei die Verarbeitungseinheit (5) des Weiteren vorgesehen ist zum:
Differenzieren von mindestens einem von der Vielzahl der Impedanzwerte der Zielgeweberegion und/oder der Referenzgeweberegion in Bezug auf Zeit, Raum, Phase und/oder Größenordnung;
Ermitteln der Größenordnung, der Phase, des Realteils und/oder des Imaginärteils von mindestens einem von der Vielzahl der Impedanzwerte der Zielgeweberegion und/oder der Referenzgeweberegion;
Ermitteln der Differenz zwischen mindestens einem von der Vielzahl der Impedanzwerte der Zielgeweberegion und mindestens einem von der Vielzahl der Impedanzwerte der Referenzgeweberegion; und/oder
Ermitteln des Kehrwerts von mindestens einem von der Vielzahl der Impedanzwerte der Zielgeweberegion und mindestens einem von der Vielzahl der Impedanzwerte der Referenzgeweberegion.

## Revendications

1. Appareil médical (10) permettant de diagnostiquer un état malade d'un tissu d'un patient, comportant :
une unité de signal d'impédance (2) étant couplée à une sonde comprenant une pluralité d'électrodes conçues pour obtenir des données d'impédance d'une région tissulaire cible au niveau de différentes couches de tissu,
dans lequel la sonde possède cinq électrodes (9a à 9e), chaque électrode (9a à 9e) comprenant au moins une pointe ou microaiguille, chaque pointe ou microaiguille ayant une longueur suffisante pour pénétrer au moins une couche de la peau d'un patient et dans lequel lesdites données d'impédance comprennent une pluralité de valeurs d'impédance mesurées dans la région tissulaire cible, au niveau de différentes couches de tissu, l'unité de signal d'impédance (2) étant en outre conçue pour obtenir des données d'impédance d'une région tissulaire de référence se trouvant au voisinage immédiat de la région tissulaire cible,
dans lequel l'unité de signal d'impédance (2) est en outre conçue pour obtenir les données d'impédance de la région tissulaire cible et les données d'impédance de la région tissulaire de référence au niveau de différentes couches de tissu sensiblement en même temps ou sensiblement consécutivement, dans lequel ladite unité de signal d'impédance (2) est conçue pour sélectionner des paires d'électrodes comprenant des électrodes adjacentes (9a à 9e) pour balayer des valeurs d'impédance au niveau d'une couche de tissu la plus supérieure et des paires d'électrodes comprenant des électrodes séparées par une ou plusieurs électrodes intermédiaires (9a à 9e) pour balayer des valeurs d'impédance au niveau de couches de tissu plus profondes sensiblement en même temps ou sensiblement consécutivement au niveau de la région tissulaire cible et de la région tissulaire de référence à la fois,
dans lequel l'unité de signal d'impédance (2) est en outre conçue pour obtenir des données d'impédance de la région tissulaire cible et/ou de la région tissulaire de référence au niveau de différentes couches de tissu, dans lequel au moins une partie supérieure du tissu est balayée de façon à obtenir une série de valeurs d'impédance depuis des petits segments tissulaires consécutifs ;
dans lequel l'impédance électrique de la région tissulaire cible et/ou de la région tissulaire de référence est mesurée à une pluralité de fréquences distribuées de manière logarithmique dans la plage d'environ 1 kHz à environ 2,5 MHz ;
une unité de classification (3) conçue pour appliquer au moins un ensemble de règles de prétraitement de données aux données d'impédance de la région tissulaire cible et à la région tissulaire de référence de façon à obtenir un ensemble de données classées pour la région tissulaire cible et un ensemble de données classées pour la région tissulaire de référence ; et
une unité de diagnostic (4) conçue pour exécuter un algorithme de système d'évaluation entraîné pour un diagnostic dudit état malade dans la région tissulaire cible sur la base de l'ensemble de données classées pour la région tissulaire cible.

2. Appareil selon la revendication 1, dans lequel l'unité de diagnostic (4) est conçue pour exécuter l'algorithme de système d'évaluation entraîné pour un diagnostic dudit état malade dans la région tissulaire cible en outre sur la base de l'ensemble de données classées pour la région tissulaire de référence.

3. Appareil selon la revendication 1, comprenant en outre une unité de traitement (5) conçue pour :
réduire la teneur en bruit dans les données d'impédance de la région tissulaire cible et/ou les données d'impédance de la région tissulaire de référence ; et/ou
réduire la dimensionnalité des données d'impédance de la région tissulaire cible et/ou des données d'impédance de la région tissulaire de référence.

4. Appareil selon la revendication 3, dans lequel l'unité de traitement (5) est en outre conçue pour :
différencier au moins une valeur de la pluralité de valeurs d'impédance de la région tissulaire cible et/ou de la région tissulaire de référence par rapport au temps, à l'espace, à la phase et/ou à la grandeur ;
déterminer la grandeur, la phase, la partie réelle et/ou la partie imaginaire d'au moins une valeur de la pluralité de valeurs d'impédance de la région tissulaire cible et/ou de la région tissulaire de référence ;
déterminer la différence entre au moins une valeur de la pluralité de valeurs d'impédance de la région tissulaire cible et au moins une valeur de la pluralité de valeurs d'impédance de la région tissulaire de référence ; et/ou
déterminer la réciproque d'au moins une valeur de la pluralité de valeurs d'impédance de la région tissulaire cible et d'au moins une valeur de la pluralité de valeurs d'impédance de la région tissulaire de référence.
